# EUROPEAN PATENT APPLICATION

(11) **EP 4 684 886 A1**
(43) Date of publication of application: **28.01.2026**
(21) Application number: 25190465.2
(22) Date of filing: 18.07.2025
(51) Int. Cl.: B06B 1/02, B06B 1/06

(54) **BACKING WITH INTEGRATED GROUND AND SIGNAL CONDUCTORS FOR AN ULTRASOUND TRANSDUCER**

(30) Priority: 23.07.2024 US 202418781935
(71) Applicant: Siemens Medical Solutions USA, Inc., Malvern, PA 19355 (US)
(72) Inventor: LEE, KyungHo, Pohang-si, Gyeomgsangbuk-do (KR); KIM, YoungShin, PoHang-si, KyungPook (KR); LEE, SeungHee, Buk-gu, Pohang-si, Gyeongsangbuk-do (KR)
(74) Representative: HKW Intellectual Property PartG mbB

(57) **Abstract**

For ultrasound transducers, signal lines and ground lines are integrated as part of the backing block. In one approach, a groove extending only partly into the backing block is filled to provide the ground path. The intervening layers (e.g., flexible circuit) between the transducer elements and backing block are replaced by the integrated signal and ground lines, so there is less energy loss and greater contrast resolution.

## Description

### BACKGROUND

The present embodiments relate to ultrasound transducers. Typical ultrasound transducers include a piezoelectric (PZT) layer stacked on a backing block. A ground sheet and a flexible circuit may be stacked between the PZT layer and the backing block. The ground sheet and flexible circuit material result in acoustic energy loss and lessens contrast resolution due to reverberation from acoustic mismatch.

### SUMMARY

By way of introduction, the preferred embodiments described below include methods, systems, backings, and components for ultrasound transducers. Signal lines and ground lines are integrated as part of the backing block. In one approach, a groove extending only partly into the backing block is filled to provide the ground path. The intervening layers (e.g., flexible circuit) between the transducer elements and backing block are replaced by the integrated signal and ground lines, so there is less energy loss and greater contrast resolution.

In a first aspect, a transducer array system is provided. A backing includes acoustic energy attenuating material, a ground conductor integrated with the acoustic energy attenuating material, and signal conductors integrated with the acoustic energy attenuating material. The ground conductor and signal conductors connect with an array of transducer elements.

In a second aspect, a backing is provided for an ultrasound transducer. A block of acoustic attenuating material is provided. A ground conductor is in a first groove of the block. A plurality of signal conductors is in the block.

In a third aspect, a method is provided for forming a backing for an acoustic transducer. A groove is formed in acoustic attenuating material. A first conductor is deposited in the groove. A ground path is connected to the first conductor. Signal lines are formed in the acoustic attenuating material. The signal lines are separate from the first conductor. Signal paths are connected to the signal lines.

Further aspects and features are summarized below in the Illustrative Embodiments. Different aspects and/or features may be used in various combinations. Aspects and/or features in one context (e.g., system, backing, or method) may be used in another context.

The present invention is defined by the following claims, and nothing in this section should be taken as a limitation on these claims. Further aspects and advantages of the invention are discussed below in conjunction with the preferred embodiments and may be later claimed independently or in combination. Different embodiments may achieve or fail to achieve different objects or advantages.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.
Figures 1A and 1B are cross-section and perspective views of an example transducer stack using a backing with integrated signal and ground lines;
Figures 2A and 2B are cross-section and perspective views of another example transducer stack using a backing with integrated signal and ground;
Figures 3A and 3B are cross-section and perspective views of yet another example transducer stack using a backing with integrated signal and ground;
Figure 4-7 are perspective views of different embodiments of a backing block with integrated signal and ground conductors; and
Figure 8 is a flow chart diagram of one embodiment of a method for forming a backing with integrated signal and conductor lines and forming a transducer with the backing.

### DETAILED DESCRIPTION OF THE DRAWINGS AND PRESENTLY PREFERRED EMBODIMENTS

A backing block structure includes signal and ground lines. This structure avoids needing intervening metal layers for the signal and ground lines between the transducer elements and the backing block. By forming grooves on the backing block and filling the grooves with conductive materials, signal and ground lines are implemented to replace the conventional signal flexible circuit and ground metal sheet. The signal lines and ground line(s) are arranged to match the distribution of elements of the array, such as a one dimensional (1D) or multi-dimensional arrangement.

The integrated ground and signal lines in the backing may reduce cost and improve acoustic performance by avoiding the performance degradation from intervening metal layers. Moreover, this backing block structure is effective in heat sinking as the piezo ceramic, which is a heat source, is directly bonded to the signal and ground lines on the backing block surface. By setting the width and depth of the ground line filled with conductive material, the acoustic and thermal design may be optimized.

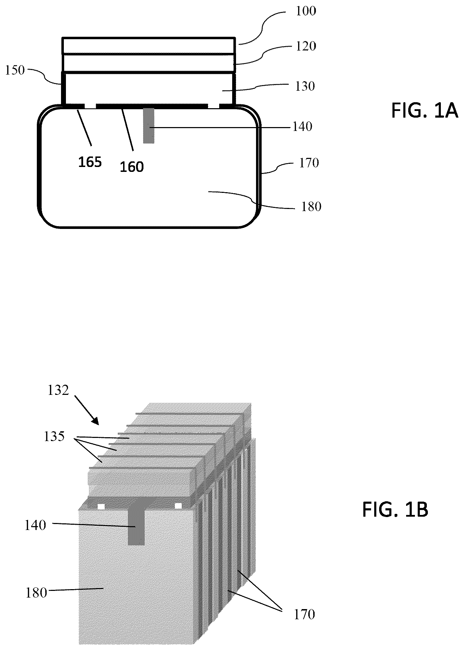

Figures 1A and 1B are a cross-section view and a perspective view of one embodiment of a transducer array system. The cross-section is in the depth-elevation dimension where depth is vertical on the drawing and elevation is horizontal on the drawing. The elements 135 of the array 132 are distributed along the azimuth dimension. The transducer array system includes a backing block 180 with integrated ground conductors 140 and signal conductors 170.

The transducer array system is used for an ultrasound transducer probe, such as in a handheld probe for scanning from an exterior of a patient or an intra-cavity (e.g., TEE or TTE) or catheter-based probe for scanning from within a patient. The system includes a one or multi-dimensional transducer array 132. As a multi-dimensional array 132, the array 132 may be a 1.25, 1.5, 1.75, or 2D array with a distribution of elements 135 in both azimuth and elevation.

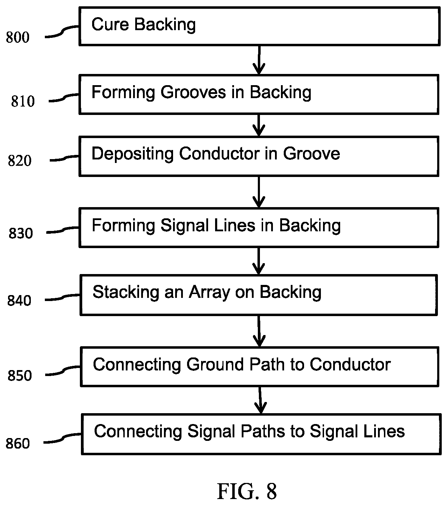

The transducer array system includes matching layers 100, 120, a wrap-around electrode150, an array layer (e.g., piezoelectric (PZT) layer 130), and an acoustic backer (backing block of acoustic attenuation material) 180. Additional, different, or fewer layers may be included, such as including integrated circuits or chips connected to the backing layer and/or not including one of the matching layers 100, 120. In some examples, a lens and/or housing is provided around the transducer array system or adjacent to the second acoustic matching layer 100. In other examples, a layer of flexible circuit material layer connects to the backing block 180 opposite the PZT layer 130. The transducer array system and corresponding probe are formed using the method of Figure 8 or another method.

The matching layers 100 and 120 are ¼ wavelength thickness layers of material. Multiple layers for a gradual change in acoustic impedance may be used, but only one matching layer is provided in other implementations. The second matching layer 100 provides a transition in acoustic impedance between the patient, lens, or other material and the first matching layer 120. Similarly, the first matching layer 120 provides an acoustic impedance transition from the second matching layer 100 to the piezoelectric or other transducer array layer 130.

The array layer is shown as a PZT layer 130 but may include the first matching layer 120. The PZT layer 130 is a slab or plate of PZT material. A solid PZT may be used. Single or poly-crystal PZT material may be used. In other embodiments, a composite of piezoelectric and epoxy or another polymer is used. Microelectromechanical (capacitive membrane) elements 135 may be used instead of PZT. Piezoelectric examples are used herein, so the array layer will be referenced as the PZT layer 130.

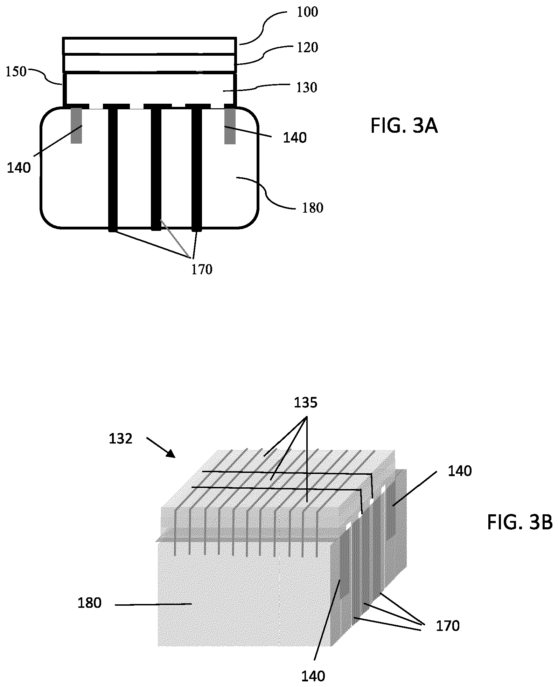

Once diced, the PZT layer 130 forms the array 132 of transducer elements 135. The transducer elements 135 of the array 132 or PZT layer 130 are distributed in a grid over one or two dimensions. As shown in Figure 1B, the PZT layer 130 may form a 1D array with the transducer elements 135 distributed in a line, whether straight or curved. Figure 3B shows a multi-dimensional array 132 where the elements 135 are distributed in two dimensions (azimuth and elevation). The multi-dimensional array 132 shown is a 1.5 or 1.75D array, but a 1.25 or 2D array may be used.

The transducer array 132 is an array of PZT elements 135. The elements 135 transduce between acoustic and electrical energies, such as an array of transducer elements 135 formed from PZT material. Kerfs separate the elements 135 of the PZT layer 130. The kerfs may also separate the first acoustic matching layer 120. In other embodiments, the kerfs separate the second matching layer 100. In yet other embodiments, the kerfs do not extend through the first acoustic matching layer 120. The array is flat, concave, or convex.

Each of the transducer elements 135 of the array includes at least two electrodes. The wrap-around electrode 150 provides one of the electrodes or is used to form both. The signal electrodes are separated by the kerfs with the PZT layer 130, such as being electrodes 165 deposited on the PZT layer 130. The elements 135 transduce between electrical and acoustical energies. The wrap-around electrode 150 or part there of defines a 0 volt or ground signal. The electrical energy generated by the PZT or provided to the PZT is provided on an electrode 165 separate from the ground portion of the wrap-around electrode 150. This signal electrode 165 is separate for each element 135, providing a separate conductive path from the PZT layer 130. The signal electrodes 165 connect with the signal conductors 170 of the backing block, forming a signal path to and from the array 132.

The wrap-around electrode 150 is a deposited conductor on the PZT layer 130 (e.g., sputter deposition or plating). The wrap-around electrode150 is deposited on the PZT layer 130 such as on a top, around the sides, and on at least part of the bottom. Kerfs are formed to separate the transducer elements 135, and, in some implementations, separate the signal electrodes and ground electrode. Alternatively, the signal electrodes are formed separately from the wrap-around electrode 150. The wrap-around electrode150 is adjacent (e.g., rests against the elements 135 and/or conductively connected to the PZT layer 130) to the acoustic array 132 on a side opposite the backing block 180. In alternative implementations, the wrap-around electrode 150 does not wrap around and/or is a sheet laid on the PZT layer 130.

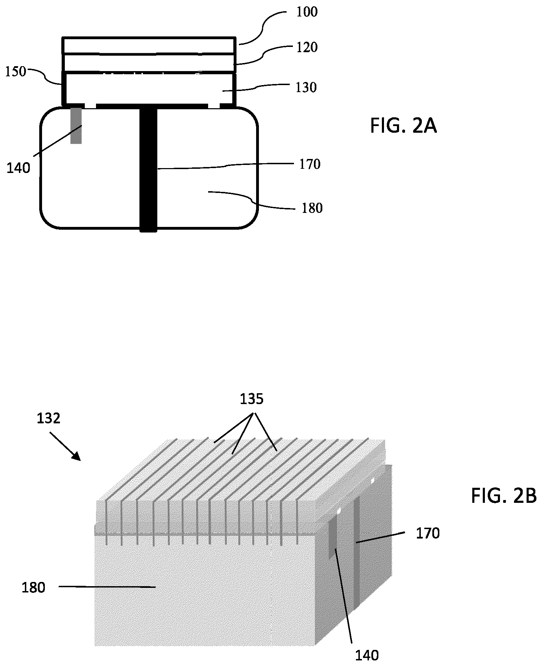

The wrap-around electrode 150 or a part thereof provides an electrode for transducing on one side of the PZT layer 130. The wrap-around electrode 150 may have traces, wires, or further sheet that extends on the sides and/or to the side of the PZT layer 130 adjacent to the backing 180. In the example of Figure 1, the wrap-around electrode 150 extends over the sides and forms the signal electrode with a separated part forming the ground connection or pad 160. The ground part of the wrap-around electrode 150 connects with the ground electrode 140 of the backing block 180 to form a ground path for the array 132. In the example of Figure 2, the wrap-around electrode 150 forms a ground electrode, with separate signal electrodes formed from part of the wrap-around electrode 150 or separately.

The array 132 and corresponding PZT layer 130 are positioned adjacent to the backing block 180. One side of the backing contacts (e.g., asperity contact or through solder) with a side of the array 132. This contact is free of a full ground sheet and free of any intervening flexible circuit material. Other layers may separate the PZT layer 130 from the backing block 180, such as bonding material.

The backing block 180 is a backing for the ultrasound transducer. The backing block 180 may be a single molded or formed (e.g., machined) block for all the elements 135. Alternatively, separate backing blocks 180 are provided for different groups of elements 135.

The backing block 180 is shaped and sized to mate with the array 132. The dimensions of the backing block 180 along azimuth and lateral dimensions of the array 132 may match the array 132 or extend beyond the array 132. The depth of the backing block 180 may be greater than the ¼ of the longest wavelength for which the array 132 is to be used. In some embodiments, the depth is greater than the depth of the PZT layer 130. The backing block 180 may include protrusions or indentations for mating with the PZT layer 130. Epoxy or other bonding agent or glue may be used to connect the backing block 180 with the PZT layer 130 in asperity contact. Clips or other structures may be added to mate or connect the backing block 180 to the array 132. Once aligned, the backing block 180 (e.g., molded block of acoustic absorber) is bonded or fixed to the PZT layer 130.

The backing block 180 is an acoustic absorber. The backing block 180 is formed from epoxy, another thermosetting or thermoplastic polymer, or another acoustic absorber. This acoustic attenuating material may be one material (e.g., cured epoxy) or a composite of different materials. The different materials may be mixed, such as a composite backing. A matrix or base material is epoxy, but other bonding agents may be used, such as elastomers, for the acoustic attenuating material. The backing block 180 is formed from a support structure of cured epoxy, such as epoxy with the thermoset and thermoplastic components mixed to chemically cure. Heat, pressure, or other environmental control may be used to form the cured epoxy. Any fillers, such as for altering the acoustic attenuation, may be mixed with the epoxy before curing.

The backing block 180 includes one or more integrated ground conductors 140. The ground conductor 140 is integrated with the acoustic energy attenuating material (e.g., epoxy block forming the backing block 180).

The backing block 180 is formed with one or more grooves. Alternatively, one or more grooves are cut or diced from the block. The groove has any shape, such as "U" or "V" shape in cross-section. The shape may vary. The width and/or depth is the same for the entire groove or varies. The depth of the groove is only partly into the backing block 180, so the groove does not extend to the opposite side from the side in which the groove is formed. The groove is on one side of the block 180 and has a depth less than a distance from that side to an opposite side. Alternatively, the groove and corresponding ground conductor 140 extend between the opposite sides.

The groove is formed on one or more sides of the backing block 180. Figures 1A and 1B show one groove on one side where the groove extends across the entire side. Lesser extents may be used.

Figures 1A and 1B show a single groove centered along an elevation dimension of the array 132. Figures 2A and 2B show a single groove extending along the azimuth dimension offset from the center in elevation. Figures 3A and 3B show two parallel grooves along an azimuth dimension and offset on opposite sides along the elevation dimension. Other placements of one groove may be used. Other arrangements of multiple grooves with parallel or non-parallel positioning may be used.

The ground conductor 140 is formed by filling the groove with conductive material. Any conductive material may be used. For example, E-solder, other solder, graphite, silver epoxy, or a metal (e.g., copper or stainless steel) is used. The conductive material flows into the groove and cures. Alternatively, the conductive material is cut to fit in the groove and then bonded (e.g., with epoxy) in the groove. The backing block 180 may be molded with or poured around the ground conductor 140 and then cured. A composite of attenuating material and the ground conductor 140 may be bonded to a side wall of other attenuating material to form the backing block 180. The integrated ground conductor 140 may replace any ground layer between the PZT layer 130 and the backing block 180.

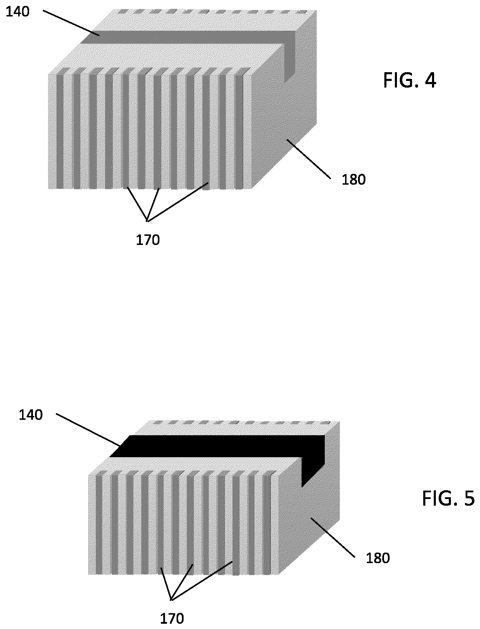

The groove and resulting ground conductor 140 may be tuned for acoustic attenuation, thermal conductivity, and/or other considerations. The width, shape, depth, and/or conductive material used may be selected to provide a desired thermal conductivity and/or acoustic attenuation. Figure 4 shows an example of the ground conductor 140 with one width and depth. Figure 5 shows another example of the ground conductor 140 that is wider and shallower.

The ground conductor 140 forms a ground path through the backing block 180 from the wrap-around electrode 150 forming the ground electrode of the array 132. Using wire bonding, asperity contact, or other electrical connection, the ground conductor 140 connects to a wire and/or system ground. The ground conductor 140 integrated in the backing block 180 forms at least part of the path to ground for the ground electrode (e.g., wrap-around electrode150) of the array 132. More than one path may be provided, such as where multiple ground conductors 140 (see Figures 3A and 3B) are used.

The ground conductor 140 replaces a sheet of metal between the array 132 and the backing block 180. The ground conductor 140 may also form a thermal path from the array 132 for heat dissipation.

The backing block 180 also integrates one or more signal conductors 170. The signal conductors 170 integrate with the acoustic energy attenuating material. A plurality of signal conductors 170 are formed, such as one or two for each transducer element 135. The signal conductors 170 connect to the signal electrodes 165 of the array 132, forming separate signal paths to and from the elements 135 for connection with the ultrasound imaging system. The signal path extends through the backing block 180 due to the signal conductors 170.

Grooves are formed for the signal conductors 170. The grooves are formed as discussed above for the ground conductor 140 or in another manner. The grooves may be vias drilled through the backing block 180, grooves cut into the backing block 180, and/or molded grooves formed when curing the backing block 180.

The grooves may be filled to form the signal conductors 170. For example, E-solder, other solder, or silver epoxy are poured or placed in grooves and cured to form the signal conductors 170. The grooves may be kerfs formed by dicing to create the signal conductors from a sheet or plate of conductive material. The kerfs may or may not be filled with epoxy.

In some implementations, the grooves and corresponding signal conductors 170 are formed on an outside of the backing block 180. Figures 1A and 1B show the signal conductors 170 on the sides. In other implementations, the grooves and corresponding signal conductors 170 are formed on an inside of the backing block 180. Figures 2A and 2B show an example. For inside signal conductors 170, the groove (e.g., via) may be filled or the epoxy cured around the signal conductors 170. The signal conductors 170 may be any of the materials used for the ground conductor 170.

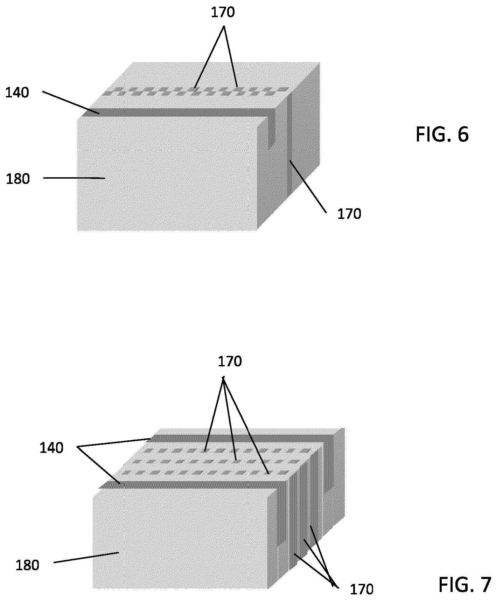

Other processes may be used to form interior signal conductors 170 in any pattern. Figure 6 shows an example. A block of acoustic attenuating material has grooves cut on two opposite sides. These grooves are filled to form signal conductors 170. The block is then cut, and the two exposed sides with signal conductors 170 are placed against each other to form the pattern of signal conductors 170 shown in Figure 6. Figure 7 shows another example. Layers of backing material with formed signal conductors 170 are stacked and bonded to form the pattern desired for the signal conductors 170. Other processes may be used, such as supporting the signal conductors 170 in a frame, filling the frame with epoxy, and curing.

The signal conductors 170 formed on the side of acoustic attenuating material may be used in that arrangement or cut from some of the material for stacking in another arrangement as the backing block 180. In one approach for forming the signal conductors 170 on a side of the backing block, a sheet of conductive material is deposited, such as depositing a sheet of gold by placing a foil or by deposition. The sheet is then diced to form the separate signal conductors 170. In another approach, a plate of conductive material is bonded to the acoustic attenuation material. The plate is then diced to form the separate signal conductors 170 as rods. As another approach, grooves are cut into the acoustic attenuation material, and the signal conductors 170 are placed in the grooves (e.g., by curing conductive fluid or bonding solid conductors in the grooves).

The signal conductors 170 extend from one side to another side of the backing block 180 in parallel. Non-parallel arrangements may be used, such as to alter a pitch of the signal conductors 170 from one pitch on the side by the array 132 to another pitch for connecting with a flexible circuit and/or integrated circuit. The signal conductors 170 may extend to sides other than the opposite side.

The signal conductors 170 electrically connect, such as through asperity contact, with the electrodes 165 of the transducer elements 135 of the array 132. The distribution of signal conductors 170 exposed on one side of the backing block 180 matches the distribution of the transducer elements 135 and/or electrodes 165 of the elements 135.

The signal conductors 170, as integrated into the backing block 180, replace conventional flexible circuit or flexible circuit material layers between the PZT layer 130 and the backing block 180. These signal conductors 170 provide a signal path as well as thermal path for conduction of heat away from the array 132.

For further connection with the ultrasound system, a flexible circuit, wire bonds, or other electrical connection extends from the ground conductor 140 and the signal conductors 170 of the backing block 180. For example, flexible circuit material with pads and metal traces are bonded to the backing block 180 opposite the array 132 in asperity contact. A wire jumper or bond connects the ground conductor 140 to the flexible circuit material or another wire. As another example, flip chip bonding or other connection is formed with an integrated circuit to the backing block 180.

Figure 8 is a flow chart of one embodiment of a method for forming a backing for an acoustic transducer and stacking with the transducer. The backing is created with ground lines and signal lines, avoiding the need for ground sheet or flexible circuit material between the array and backing in the transducer.

The method forms the array system of Figures 1A, 1B, 2A, 2B, 3A, 3B, or another array system. The method forms the backing of Figures 1-7 or another backing. The method is implemented as a manufacturing of the array system and/or backing. A technician or robot stacks and aligns, such as using guideposts or a frame. An oven, iron, induction solderer, press, and/or wave bath is used to bond or interconnect. A frame, housing, clamp, or holder are used to shape and position in a probe housing.

Additional, different, or fewer acts may be used. For example, act 800 is not provided where a pre-made initial block of acoustic attenuation material is provided. As another example, acts 840, 850, and/or 860 are not provided. As yet another example, act 810 is not provided where the groove is formed as part of curing in 800. In another example, acts for adding matching layers, adding a lens, dicing, adding other probe components, and/or other manufacturing (e.g., grinding) are provided. In yet another example, testing of the components or parts, subassembly, and/or entire assembly is provided.

The acts are performed in the order shown (top to bottom or numerical) or other orders. For example, acts 830, 820, and/or 800 may be performed at a same time. As another example, acts 850 and/or 860 are performed prior to act 840 and in any order relative to each other.

In act 800, the backing is cured. The epoxy or other acoustic attenuating material is poured or injected into a mold. Through chemical reaction with or without application of heat and/or pressure, the backing cures. The result is a block shaped and sized for the array or shaped and sized for cutting to the array. The block may be ground or cut for further shaping and/or sizing.

The cured block or acoustic attenuation material may have flat, planar surfaces and/or curved surfaces without grooves for the ground and/or signal lines. The cured block or backing may include one or more grooves for conductors. For example, the mold includes a groove for the ground conductor and/or grooves for the signal conductors.

In act 810, one or more grooves are formed in the acoustic attenuating material. A saw or grinder cuts the grooves on one or more outer surfaces of the block. Alternatively, a drill cuts the groove through an interior of the block as a via.

For the forming the signal lines in act 830, the grooves may be formed in act 810 as kerfs by dicing. For example, gold plating or other conductive metal is deposited as a sheet on a surface of the block. Sputter deposition or bonding a foil may be used. The sheet is diced, separating the sheet into separate signal conductors. The grooves are kerfs between the signal lines. In another example, a plate of conductive material (e.g., graphite) is bonded to the block. The plate is then diced, separating the plate into separate signal conductors. The grooves are kerfs between the signal lines. As another example, grooves are cut in the attenuating material. These grooves are then filled, such as by adding a fluid or placing a solid. The fill material in the grooves is conductive so forms the signal lines.

The grooves are formed at the desired pitch. In one approach, the pitch is the same as the elements of the array. A greater pitch may be used, such as where multiple signal conductors are to connect to each element.

For depositing the conductor for grounding in the groove in act 820, the groove is formed in act 810 to connect with the ground of each element and/or the array. For example, the groove extends along azimuth of a one, 1.25, 1.5, or 1.75D array. The groove may extend from one side to another side of the side of the block to connect with the array. A shorter extent may be provided. The depth of the groove is less than the entire depth of the block but may be through the block in other approaches.

The width and/or depth of the groove or grooves for the ground conductor(s) may be tuned or optimized for attenuation and/or thermal conductivity. For example, smaller width may result in less reverberation and greater attenuation. A larger width may provide a greater thermal conduction path to help cool the array. These considerations, with or without other considerations (e.g., cost of conductive material), are balanced to establish the width and/or depth of the groove.

In act 820, a conductor is deposited in the groove or grooves used for the grounding path. For example, the groove is filled with E-solder, other solder, or silver epoxy. Other fluid conductors may be deposited. The fluid conductor is cured or solidifies in the groove. Excess may be ground off. As another example, a solid conductor sized for the groove is placed in the groove and bonded into the groove. In another example, a surface sheet or plate is cut to form the ground conductor, and attenuation material is added beside the ground conductor to form the groove. Other techniques to fill the groove with conductive material may be used.

In act 830, signal lines are formed in the acoustic attenuating material. The signal lines are separate from each other and the ground conductors. Using the same or different approaches for depositing the ground conductor, the signal conductors are formed.

The signal lines are formed at a desired pitch based on the elements of the array. The distribution of signal lines at a surface of the backing to contact the array matches a distribution of elements of the array. For a 1D or 1.25D array, the signal lines may be formed just on one or more outside surfaces of the backing. For any array, the signal lines may be formed as vias in the backing or a combination of vias and lines on an outside surface.

In one approach, the signals lines are formed on an outside surface. The block is then cut at any desired depth from the signal lines, forming one or more slabs with signal conductors. By stacking and bonding such slabs, some or all the signal conductors are then in the backing block, such as shown in Figures 2A, 2B, 3A, 3B, 6, or 7. In the example of Figure 6, the block may be halved, the two halves flipped so that the signal conductors 170 are adjacent each other instead of opposite each other. The halves are then bonded. In the example of Figure 7, different slabs of the same or different width are cut off after forming the signal lines, and then the slabs are stacked to provide the distribution of signal lines 170. Alternatively, vias are drilled into the block as interior grooves at the desired pattern. The vias are then filled with conductive material.

The signal lines and ground lines are integrated in the backing. The integration provides for the signal lines and/or ground lines on a surface and/or through an interior of the backing. The signal lines and/or ground lines may not extend beyond a surface of the backing, such as by depositing attenuation material and planing. Alternatively, the signal lines and/or ground line may extend from the attenuation material while being integrated with or part of the backing block. The signal and ground lines are formed on or in the backing block rather than using separate layers for a transducer probe.

In act 840, the array of transducer elements is stacked on the backing block. The array may include matching layers, a ground plane, electrodes, and/or other components. The components as bonded together are stacked on the backing. Alternatively, the different layers are stacked in sequence with other processing, such as stacking the PZT with deposited conductors and a matching layer on the backing, bonding, then dicing, and then stacking another matching layer and bonding.

Prior to stacking or after stacking, the array is formed by dicing the PZT layers and signal electrodes. The resulting transducer elements are distributed in one or two dimensions. The elements and/or signal electrodes of the elements are aligned with the signal conductors in the backing block of acoustic attenuating material. The stacking aligns to provide asperity or solder contact for an electrical path from the elements through the signal lines. Similarly, the ground electrode of the array stack is aligned to contact or solder to the ground conductors in the backing, providing a ground path from the array through the backing block.

As part of stacking, the layers are bonded together. Epoxy or other adhesive bonds the layers together. The result is a transducer formed by an array of elements and acoustic attenuation material behind the array. The integrated signal and ground lines of the acoustic attenuation material are used for electrical signaling to and from the array to and from the ultrasound imaging system.

In act 850, the ground path is connected to the ground conductor of the backing. Wire bonding, soldering, asperity contact, or other conductive connection is formed from a probe or system ground to the ground line in the backing, grounding the array. A wire, trace of flexible circuit or printed circuit board, or other conductor is electrically connected with the ground conductor of the backing block.

In act 860, the signal paths for the probe or ultrasound system are connected with the signal lines in the backing. Any of the connections discussed for act 850 may be used. For example, channel or signal lines of a probe cable connect to the signal lines in the backing through traces on flexible circuit material bonded in asperity contact with a bottom (side opposite the array) surface of the backing block.

Once the ground and signal lines are connected in acts 850 and 860, the array may be used for ultrasound scanning or imaging. The ground and signal paths, including parts through the backing block, are used for imaging.

Below are Illustrative Embodiments. These Illustrative Embodiments summarize various aspects or features. The different Illustrative Embodiments may be combined as provided below or in other combinations. Aspects or features of one type (e.g., system, backing, or method) may be combined with or used with another type.

Illustrative Embodiment 1. A transducer array system comprising: an array of transducer elements; and a backing comprising acoustic energy attenuating material, a ground conductor integrated with the acoustic energy attenuating material, and signal conductors integrated with the acoustic energy attenuating material, the ground conductor and signal conductors connected with the array.

Illustrative Embodiment 2. The transducer array system of Illustrative Embodiment 1 wherein the array comprises a one-dimensional array with the transducer elements distributed in a line.

Illustrative Embodiment 3. The transducer array system of any of Illustrative Embodiments 1-2 wherein the array comprises a ground electrode connected with the ground conductor, and wherein the ground conductor forms a ground path through the backing from the ground electrode.

Illustrative Embodiment 4. The transducer array system of any of Illustrative Embodiments 1-3 wherein the array comprises signal electrodes connected with the signal conductors, and wherein the signal conductors form a signal path through the backing from the signal electrodes.

Illustrative Embodiment 5. The transducer array system of any of Illustrative Embodiments 1-4 wherein the array connects to the backing without intervening flexible circuit material.

Illustrative Embodiment 6. The transducer array system of any of Illustrative Embodiments 1-5 wherein the acoustic energy attenuating material comprises thermosetting or thermoplastic polymers.

Illustrative Embodiment 7. The transducer array system of any of Illustrative Embodiments 1-6 wherein the array is positioned adjacent a first side of the backing, the ground conductor is in a groove on the first side of the backing without extending to a second opposite side of the backing.

Illustrative Embodiment 8. The transducer array system of Illustrative Embodiment 7 wherein the groove has a width on the first side and a depth from the first side, the width and depth tuned for thermal conductivity.

Illustrative Embodiment 9. The transducer array system of claim 1 wherein the array is positioned adjacent a first side of the backing, the signal conductors extending from the first side to a second side of the backing, the second side opposite to the first side.

Illustrative Embodiment 10. The transducer array system of any of Illustrative Embodiments 1-9 wherein the signal conductors comprise a diced sheet of deposited conductor material.

Illustrative Embodiment 11. The transducer array system of any of Illustrative Embodiments 1-10 wherein the signal conductors comprise conductive material deposited in grooves in the acoustic attenuating material.

Illustrative Embodiment 12. The transducer array system of any of Illustrative Embodiments 1-11 wherein the signal conductors comprise a plate of conductive material separated into rods.

Illustrative Embodiment 13. The transducer array system of any of Illustrative Embodiments 1-12 wherein the array comprises a multi-dimensional array with the transducer elements distributed in two dimensions, wherein the signal conductors have a distribution in the backing matching the distribution of the transducer elements, and wherein the ground conductor comprises multiple ground conductors in the backing.

Illustrative Embodiment 14. A backing for an ultrasound transducer, the backing comprising: a block of acoustic attenuating material; a ground conductor in a first groove of the block; and a plurality of signal conductors in the block.

Illustrative Embodiment 15. The backing of Illustrative Embodiment 14 wherein the first groove is on a first side of the block.

Illustrative Embodiment 16. The backing of any of Illustrative Embodiments 14-15 wherein the signal conductors comprise diced rods, diced sheet, or filled vias of conductive material through the block.

Illustrative Embodiment 17. A method for forming a backing for an acoustic transducer, the method comprising: forming a groove in acoustic attenuating material; depositing a first conductor in the groove; connecting a ground path to the first conductor; forming signal lines in the acoustic attenuating material, the signal lines separate from the first conductor; and connecting signal paths to the signal lines.

Illustrative Embodiment 18. The method of Illustrative Embodiment 17 wherein forming the groove comprises forming the groove with a width and/or a depth based, at least in part, on thermal conductivity, the depth of the groove being less than a depth of the acoustic attenuating material.

Illustrative Embodiment 19. The method of any of Illustrative Embodiments 17-18 wherein forming the signal lines comprises: (a) depositing a sheet of conductive material and dicing the sheet; (b) connecting a plate of conductive material and dicing the plate; or (c) cutting vias through the acoustic attenuating material and filling the vias with conductive material.

Illustrative Embodiment 20. The method of any of Illustrative Embodiments 17-19 further comprising: stacking an array of transducer elements distributed in two dimensions on the acoustic attenuating material; wherein forming the signal lines comprises forming the signal lines with a distribution in the two dimensions matching the distribution of the transducer elements.

Independent of the grammatical term usage, individuals with male, female or other gender identities are included within the term.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A transducer array system comprising:
an array (132) of transducer elements (135); and
a backing (180) comprising acoustic energy attenuating material, a ground conductor (140) integrated with the acoustic energy attenuating material, and signal conductors (170) integrated with the acoustic energy attenuating material, the ground conductor (140) and signal conductors (170) connected with the array (132).

2. The transducer array system of claim 1 wherein the array (132) comprises a one-dimensional array (132) with the transducer elements (135) distributed in a line, and/orwherein the array (132) comprises a ground electrode (160) connected with the ground conductor (140), and wherein the ground conductor (140) forms a ground path through the backing (180) from the ground electrode (160).

3. The transducer array system of claim 1 or 2 wherein the array (132) comprises signal electrodes (165) connected with the signal conductors (170), and wherein the signal conductors (170) form a signal path through the backing (180) from the signal electrodes (165), and/or wherein the array (132) connects to the backing (180) without intervening flexible circuit material.

4. The transducer array system of one of claims 1 to 3 wherein the acoustic energy attenuating material comprises thermosetting or thermoplastic polymers.

5. The transducer array system of one of claims 1 to 4 wherein the array (132) is positioned adjacent a first side of the backing (180), the ground conductor (140) is in a groove on the first side of the backing (180) without extending to a second opposite side of the backing (180), wherein the groove particularly has a width on the first side and a depth from the first side, the width and depth tuned for thermal conductivity.

6. The transducer array system of one of claims 1 to 5 wherein the array (132) is positioned adjacent a first side of the backing (180), the signal conductors (170) extending from the first side to a second side of the backing (180), the second side opposite to the first side.

7. The transducer array system of one of claims 1 to 6 wherein the signal conductors (170) comprise a diced sheet of deposited conductor material, and/or wherein the signal conductors (170) comprise conductive material deposited in grooves in the acoustic attenuating material.

8. The transducer array system of one of claims 1 to 7 wherein the signal conductors (170) comprise a plate of conductive material separated into rods.

9. The transducer array system of one of claims 1 to 8 wherein the array (132) comprises a multi-dimensional array (132) with the transducer elements (135) distributed in two dimensions, wherein the signal conductors (170) have a distribution in the backing (180) matching the distribution of the transducer elements (135), and wherein the ground conductor (140) comprises multiple ground conductors (140) in the backing (180).

10. A backing for an ultrasound transducer, the backing comprising:
a block (180) of acoustic attenuating material;
a ground conductor (140) in a first groove of the block (180); and
a plurality of signal conductors (170) in the block (180).

11. The backing of claim 10 wherein the first groove is on a first side of the block (180).

12. The backing (180) of claim 10 or 11 wherein the signal conductors (170) comprise diced rods, diced sheet, or filled vias of conductive material through the block (180).

13. A method for forming a backing for an acoustic transducer, the method comprising:
forming (810) a groove in acoustic attenuating material;
depositing (820) a first conductor in the groove;
connecting (850) a ground path to the first conductor;
forming (830) signal lines in the acoustic attenuating material, the signal lines separate from the first conductor; and
connecting (860) signal paths to the signal lines.

14. The method of claim 13 wherein forming (810) the groove comprises forming (810) the groove with a width and/or a depth based, at least in part, on thermal conductivity, the depth of the groove being less than a depth of the acoustic attenuating material.

15. The method of claim 13 or 14 wherein forming (830) the signal lines comprises:
(a) depositing a sheet of conductive material and dicing the sheet;
(b) connecting a plate of conductive material and dicing the plate; or
(c) cutting vias through the acoustic attenuating material and filling the vias with conductive material,
particularly further comprising:
stacking (840) an array (132) of transducer elements (135) distributed in two dimensions on the acoustic attenuating material;
wherein forming (830) the signal lines comprises forming (830) the signal lines with a distribution in the two dimensions matching the distribution of the transducer elements (135).
